(19) Europäisches Patentamt
European Patent Office
Office européen des brevets

(11) Publication number: **0 071 682**
**B1**

(12) # EUROPEAN PATENT SPECIFICATION

(45) Date of publication of patent specification: **13.11.85**

(51) Int. Cl.$^4$: **C 07 C 101/54,** A 61 K 31/245

(21) Application number: **81303674.6**

(22) Date of filing: **12.08.81**

(54) **New anthranilic acid esters having anti-inflammatory activity and their preparation.**

(43) Date of publication of application:
**16.02.83 Bulletin 83/07**

(45) Publication of the grant of the patent:
**13.11.85 Bulletin 85/46**

(84) Designated Contracting States:
**DE FR GB**

(58) References cited:
**DE-A-2 914 935**

(73) Proprietor: **Council of Scientific and Industrial Research**
**Rafi Marg**
**New Delhi 110 001 (IN)**

(72) Inventor: **Sattur, Pralhad Balvantrao**
**3/4/1013-5, Rajendra Colony**
**Barkatpura, 500 027 Andhra Pradesh (IN)**
Inventor: **Bhanumathi, Nanduri**
**Z.P. 1-316 Tarnaka**
**Hyerabad - 500 017 Andhra Pradesh (IN)**

(74) Representative: **Collier, Jeremy Austin Grey et al**
**J.A.Kemp & Co. 14, South Square Gray's Inn**
**London WC1R 5EU (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to anthranilic acid esters of pharmacological interest and their preparation.

The anthranilic acid esters of the present invention, the structure of which has been confirmed by elemental analysis and spectroscopic evidence, have the general formula:

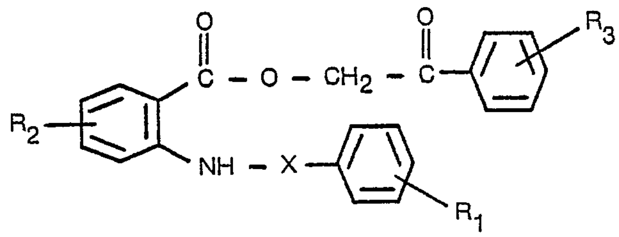

(I)

wherein $R_1$, $R_2$ and $R_3$, which may be the same or different, are each hydrogen, chlorine, methoxy or methyl, and X is a straight or branched alkylene chain having from 1 to 4 carbon atoms. These compounds show activity in the suppression of inflammation and are useful as anti-inflammatory agents, blood platelet aggregation inhibitors and prostaglandin synthetase inhibitors.

Specific compounds of the present invention are:

N-β-phenylethyl-phenacyl anthranilate of the formula:

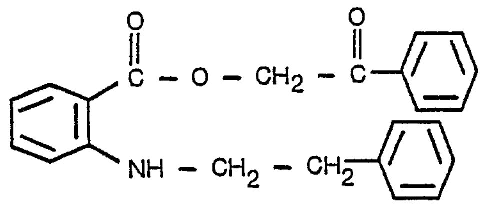

N-β-phenylethyl-4-methoxy phenacyl anthranilate of the formula:

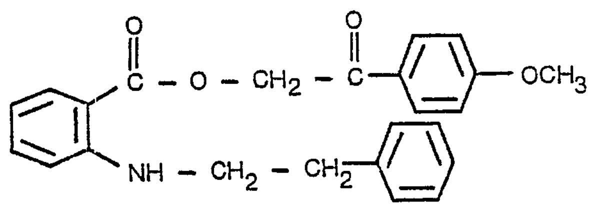

N-β-phenylethyl-4′-methyl phenacyl anthranilate of the formula:

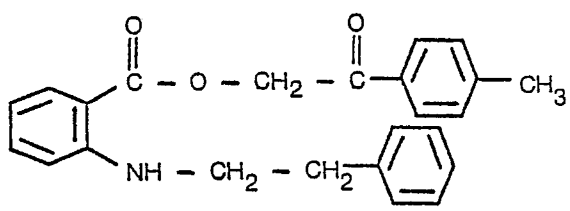

, and

N-β-phenylethyl-4′-chlorophenacyl anthranilate of the formula:

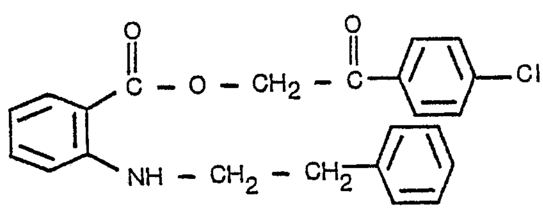

The present invention also provides a process for the preparation of anthranilic acid esters of the general formula I which comprises reacting an alkali metal salt of a compound of the general formula:

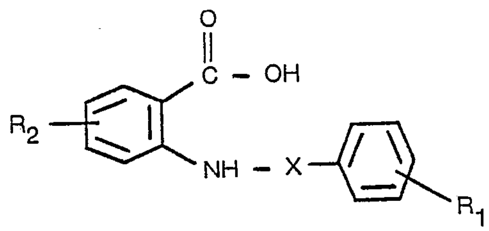

II

wherein $R_1$, $R_2$ and X have the meanings given above with a compound of the general formula:

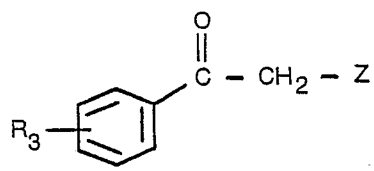

III

wherein $R_3$ has the meaning given above and Z is a halogen atom, the reaction being preferably effected in the presence of a solvent.

Preferably, the alkali metal salt employed as reactant is the sodium or potassium salt while the preferred solvent is an aliphatic ketone such as acetone, or a higher ketone.

The reaction mixture is preferably refluxed at or near the boiling point of the solvent to obtain separation of the desired esters. The refluxing is usually effected for from 60 to 240 minutes.

The preparation of the novel esters of the invention is illustrated in the following Examples:

Example I

N-β-phenylethyl-phenacyl anthranilate

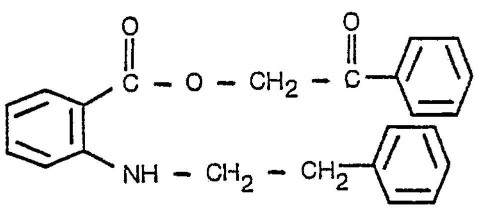

15 grams of the sodium salt of N-β-phenylethyl anthranilic acid were taken in a round-bottomed flask fitted with a condenser. To this were added 8 grams of ω-chloroacetophenone in 85 ml of acetone. The reaction mixture was refluxed for about 4 hours. After removal of the solvent, the mixture was poured into ice water and filtered. The residue was recrystallised from ethanol. Yield 85% of the desired product; m.p. 120°C.

Example II

N-β-phenylethyl-4′-methoxyphenacyl anthranilate

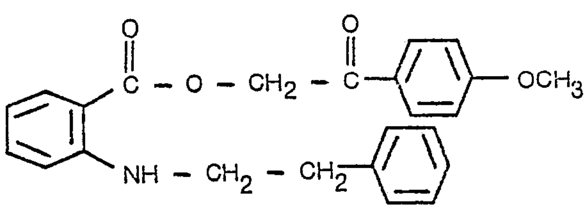

Following the procedure of Example I, 15 grams of sodium N-β-phenylethyl anthranilate were reacted with 12 grams of 4-methoxy-ω-bromoacetophenone in 100 ml of acetone. The reaction mixture was refluxed for 200 minutes after which the solvent was removed and the mixture poured into ice water, and filtered. Recrystallisation of the residue from ethanol gave a yield of 80% of the desired product, m.p. 103°C.

Example III

N-β-phenylethyl-4′-methylphenacyl anthranilate

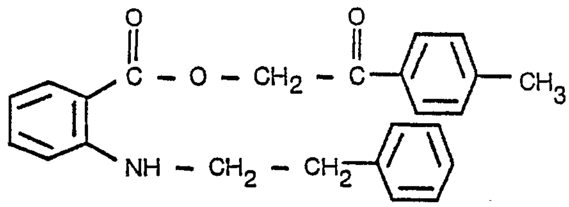

The same procedure as in Examples I and II was followed by refluxing for 180 minutes 15 grams of sodium N-β-phenylethyl anthranilate in a solution of 11 grams of 4′-methyl-ω-chloroacetophenone in 100 ml of acetone. After removal of the solvent, the mixture was poured into ice water, and filtered. The residue was recrystallised from ethanol to give a yield of 80% of the desired product, m.p. 105°C.

Example IV

N-β-phenylethyl-4′-chlorophenylacyl anthranilate

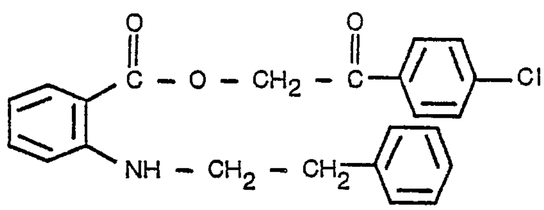

The procedure of the earlier Examples was followed using 15 grams of sodium N-β-phenylethyl anthranilate and 12 grams of 4′-chlorobromoacetophenone dissolved in 120 ml of acetone. After refluxing for 180 minutes, the solvent was removed and the mixture poured into ice water and filtered. Recrystallisation of the residue from ethanol gave a yield of 80% of the desired product, m.p. 115°C.

The pharmaceutical activity of the compounds of the invention has been studied and the results of this study in respect of 2-N-β-phenylethyl phenacyl anthranilate, which are typical of the results of tests carried out with other compounds of the invention, are set out below.

N-β-phenylethyl phenacyl anthranilate has an $LD_{50}$ of more than 800 mg/kg i.p. mice and shows potent anti-inflammatory activity as observed from the following tests.

*Anti-inflammatory Action in Rats:* Rats in which inflammatory symptoms had been induced by injections of carrageenin were treated with N-β-phenylethyl phenacyl anthranilate. Alternatively, rats treated with N-β-phenylethyl phenacyl anthranilate were then injected with carrageenin. In either instance, the dose was 100 mg/kg p.o. The following effect were observed:

(i) 44.5% inhibition of inflammation when the dose was given one hour before the injection of carrageenin.

(ii) 30% inhibition of inflammation when the dose was given half an hour before the injection of carrageenin.

(iii) 43% inhibition of inflammation when the dose was given half an hour after the injection of carrageenin.

*Carrageenin Oedema in Rats:*

The test was similar to the one for anti-inflammatory action except that the dose of N-β-phenylethyl phenacyl anthranilate was 200 mg/kg p.o. The following effects were observed:

(i) 60% inhibition of oedema when the dose was given one hour before the injection of carrageenin.

(ii) 43% inhibition of oedema when the dose was given half an hour before the injection of carrageenin.

(iii) 62% inhibition of oedema when the dose was given half an hour after the injection of carrageenin.

*Formaldehyde Arthritis:*

A daily dose of 100 mg/kg administered orally for 10 days produced 14% inhibition of formaldehyde-induced arthritis in rats.

*Cotton Pellet Granuloma:*

A daily dose of 100 mg/kg administered orally for 7 days produced a 31% inhibition of cotton pellet granuloma in rats.

*Mouse Capillary Permeability:*

At 100 mg, the dose produced 52% inhibition.

*Ulcerogenic Properties:*

Examination of rats to whom a daily dose of 100 mg/kg of N-β-phenylethyl phenacyl anthranilate had been administered orally for seven days revealed no ulcerogenic activity.

**Claims**

1. Anthranilic acid esters of the formula:

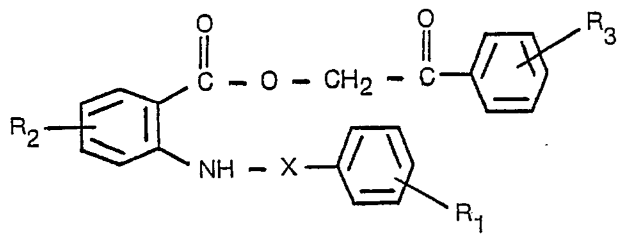

wherein $R_1$, $R_2$ and $R_3$, which may be the same or different, are each hydrogen, chlorine, methoxy or methyl, and X is a straight or branched alkylene chain having from 1 to 4 carbon atoms.

2. N-β-phenylethyl-phenacyl anthranilate of the formula:

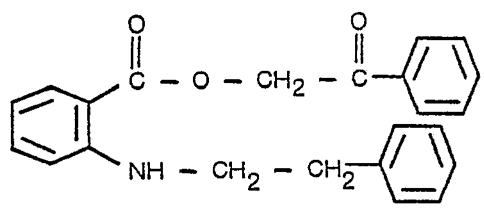

3. N-β-phenylethyl-4′-methoxyphenacyl anthranilate of the formula:

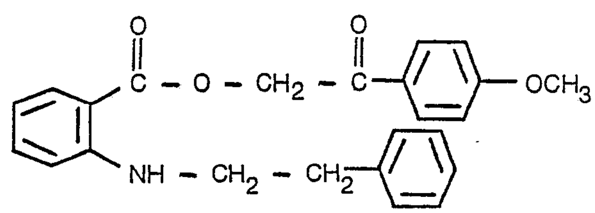

4. N-β-phenylethyl-4′-methylphenacyl anthranilate of the formula:

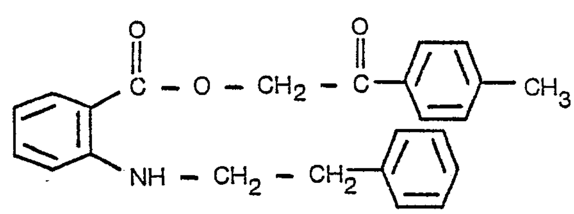

5. N-β-phenylethyl-4′-chlorophenacyl anthranilate of the formula:

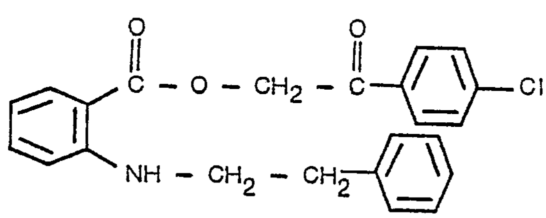

6. A process for the preparation of an anthranilic acid ester as defined in Claim 1 which comprises reacting an alkali metal salt of a compound of the formula:

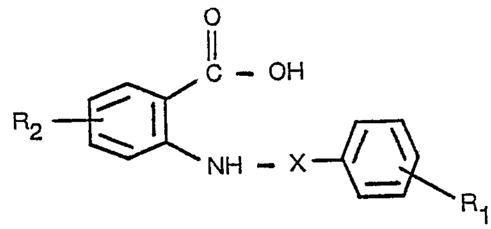

wherein $R_1$, $R_2$ and X are as defined in Claim 1 with a compound of the formula:

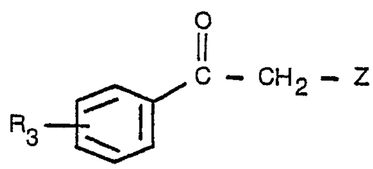

wherein $R_3$ is as defined in Claim 1 and Z is a halogen atom.

7. A process as claimed in Claim 6, wherein the reaction is effected in an aliphatic ketone.

8. An anthranilic acid ester as claimed in any one of Claims 1 to 5 for use in therapy as an anti-inflammatory agent, blood platelet aggregation inhibitor, or prostaglandin synthetase inhibitor.

**Patentansprüche**

1. Anthranilsäureester der Formel:

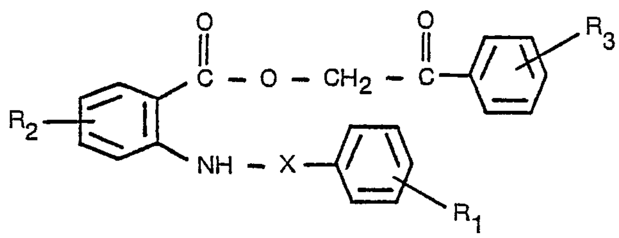

in der $R_1$, $R_2$ und $R_3$, die gleich oder verschieden sein können, jeweils Waserstoff, Chor, Methoxy oder Methyl sind und X eine geradkettige oder verzweigtkettige Alkylenkette mit 1 bis 4 Kohlenstoffatomen ist.

2. N-β-Phenylethyl-phenyacylanthranilat der Formel:

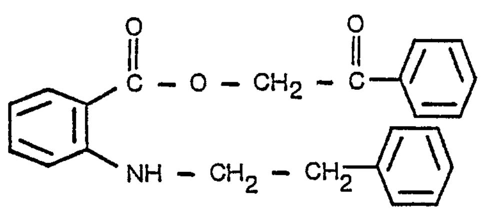

3. N-β-Phenylethyl-4′-methoxyphenacylanthranilat der Formel:

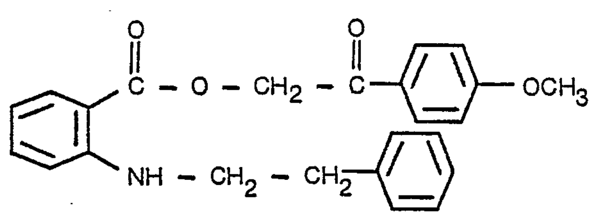

4. N-β-Phenylethyl-4'-methylphenacylanthranilat der Formel:

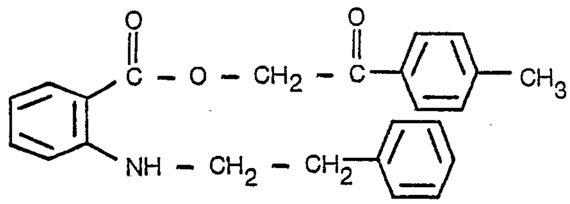

5. N-β-Phenylethyl-4'-chlorphenacylanthranilat der Formel:

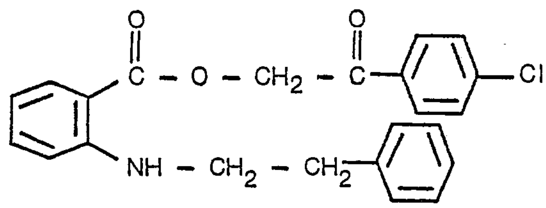

6. Verfahren zur Herstellung eines Anthranilsäureesters gemäß der Definition in Anspruch 1, das dadurch gekennzeichnet ist, daß man ein Alkalimetallsalz einer Verbindung mit der Formel:

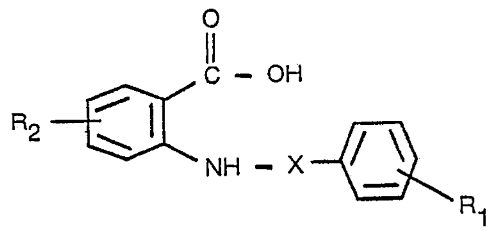

in der $R_1$, $R_2$ und X die in Anspruch 1 angegebene Bedeutung besitzen, mit einer Verbindung der Formel:

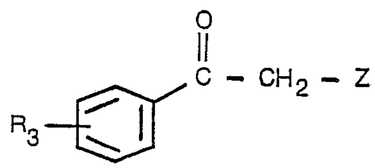

in der $R_3$ die in Anspruch 1 angegebene Bedeutung besitzt, und Z ein Halogenatom ist, umsetzt.

7. Verfahren nach Anspruch 6, dadurch gekennzeichnet, daß die Reaktion in einem aliphatischen Keton erfolgt.

8. Anthranilsäureester gemäß einem der Ansprüche 1 bis 5 zur Verwendung in der Therapie als entzündungshemmendes Mittel, Mittel gegen Thrombocytenaggregation oder Prostaglandinsyntheaseinhibitor.

**Revendications**

1. Esters d'acide anthranilique de formule:

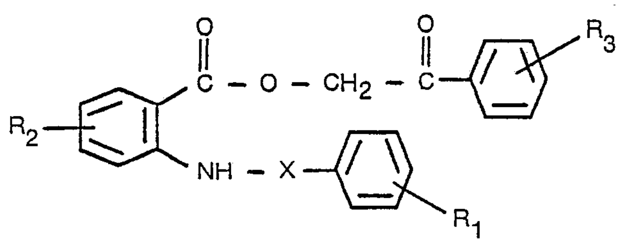

dans laquelle $R_1$, $R_2$ et $R_3$, qui peuvent être identique ou différents, représentent chacun l'hydrogène, le chlore, un groupe méthoxy ou méthyle, et X une chaîne alkylène linéaire ou ramifiée ayant de 1 à 4 atomes de carbone.

2. N-β-phényléthyl-anthranilate de phénacyle de formule:

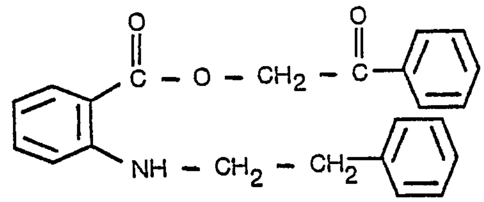

3. N-β-phényléthyl-anthranilate de 4'-méthoxyphénacyle de formule:

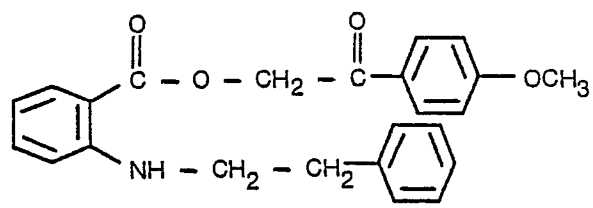

4. N-β-phényléthyl-anthranilate de 4'-méthylphénacyle de formule:

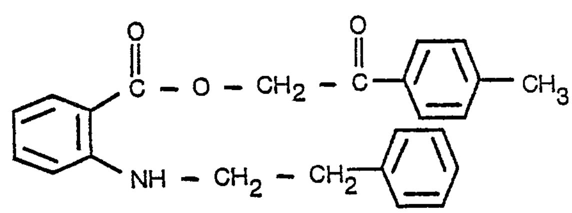

5. N-β-phényléthyl-anthranilate de 4'-chlorophénacyle de formule:

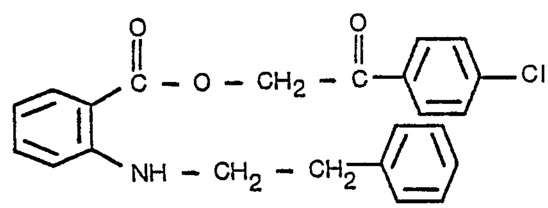

6. Procédé de préparation d'un ester d'acide anthranilique tel que défini à la revendication 1, qui consiste à faire réagir un sel de métal alcalin d'un composé de formule:

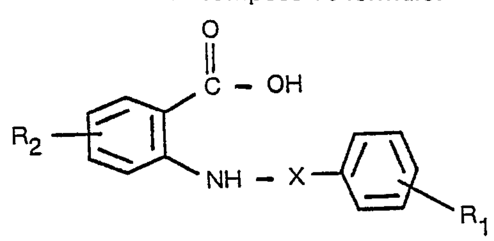

dans laquelle $R_1$, $R_2$ et X sont tels que définis à la revendication 1 avec un composé de formule:

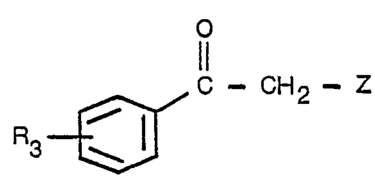

dans laquelle $R_3$ est tel que défini à la revendication et Z est un atome d'halogène.

7. Procédé selon la revendication 6, dans lequel on effectue la réaction dans une cétone aliphatique.

8. Ester d'acide anthranilique selon l'une des revendications 1 à 5, destiné à être utilisé en thérapie en tant qu'agent anti-inflammatoire, inhibiteur d'agrégation plaquettaire sanguine ou inhibiteur de prostaglandine synthétase.